# EUROPEAN PATENT APPLICATION

(11) **EP 4 516 294 A1**
(43) Date of publication of application: **05.03.2025**
(21) Application number: 23795673.5
(22) Date of filing: 04.05.2023
(51) Int. Cl.: A61K 9/22, A61K 9/24, A61K 31/4418, A61P 11/00

(54) **PIRFENIDONE SUSTAINED-RELEASE ORAL SOLID PREPARATION**

(30) Priority: 29.04.2022 CN 202210477923
(71) Applicant: Overseas Pharmaceuticals, Ltd., China 510530 (CN)
(72) Inventor: SI, Junren, Guangzhou, Guangdong 510530 (CN)
(74) Representative: Murgitroyd & Company
(86) International application number: PCT/CN2023/091991
(87) International publication number: WO 2023/208241

(57) **Abstract**

The present invention provides "a prolonged-release oral solid formulation of pirfenidone", and relates to a formulation technology. The formulation includes a sustained-release formulation. The sustained-release formulation contains a swelling material, a shaping material and an adhesive material. The weight percentage of the swelling material in the solid formulation is 10-25%; the weight percentage of the shaping material in the solid formulation is 10-25%; and the weight percentage of the adhesive material in the solid formulation is 1-8%. The solid formulation only needs to be taken once a day, regardless of whether it is taken before or after meals. It takes effect quickly after taking it, and can be stably and effectively released for 24 hours, providing a pirfenidone product with significantly improved compliance and stable blood drug concentration.

## Description

### TECHNICAL FIELD

The present invention belongs to pharmaceutical technology, and particularly relates to a prolonged-release oral solid formulation of pirfenidone.

### BACKGROUND

The existing pirfenidone products on the market are immediate-release tablets and capsules. There is no once-daily prolonged-release formulation on the market. The existing dosage form varieties have the following disadvantages:
1. the existing products require administration three times a day, and has poor patient compliance.
   The existing products are all ordinary immediate-release formulations. Their clinical data and instructions clearly state that the products need to be taken after meals, and have low patient compliance. If the patients forget how to take the formulations and thus take them before meals, adverse responses may be easily caused.
2. The medication mode of the existing products is affected by meal times, and the effective blood drug concentration is lower at night.
   The existing products are all ordinary immediate-release formulations, and their clinical data and instructions clearly state that the products need to be taken after meals. Therefore, the daily meal and medication times are fixed at 08:00, 12:00, and 18:00, but this series of medication times will result in higher blood drug concentrations during the day and too low blood drug concentrations at night, making it difficult to truly achieve a stable and effective blood drug concentration within 24 h.
3. Existing data shows that the adverse responses of this variety are related to the maximum blood drug concentration Cₘₐₓ.
   The existing products are all immediate-release formulations, which will undergo the process of rapid disintegration, release and absorption into the blood after being ingested by the human body. Rapid absorption makes that the time Tₘₐₓ for the maximum blood drug concentration is only about 1-2 h, and the half-life T_{1/2} is about 2 h, resulting in the rapid appearance of the maximum blood drug concentration Cₘₐₓ in the body. Dosing three times a day means that the maximum blood drug concentration Cₘₐₓ will appear for three times. A large amount of literature has shown that excessively high C ₘₐₓ of this drug causes a series of adverse responses, and the original research reduces the adverse responses caused by excessively high Cₘₐₓ by recommending post-meal administration. Therefore, the series of adverse responses caused by higher C ₘₐₓ after multiple daily dosing are not truly improved.
4. The existing products have large peak-to-valley ratios (Cₘₐₓ/Cₘᵢₙ) and unstable blood drug concentrations.

The existing products are administered three times a day. The frequent dosing results in a large difference between peak and valley blood drug concentrations, a large peak-to-valley ratio (Cₘₐₓ/Cₘᵢₙ), and a large number of fluctuations, making it impossible to form a stable blood drug concentration. According to common sense in the pharmaceutical field, a larger peak-to-valley ratio is more likely to cause a series of adverse responses.

To sum up, it is necessary to research and develop a novel dosage form product of pirfenidone with good compliance, reduced frequency of dosing and stable fluctuation of blood drug.

### SUMMARY

Aiming at the problems that the existing product has poor compliance due to dosing for multiple times a day, and the blood concentration of the existing product fluctuates greatly and is not stable, the present invention develops an oral prolonged-release formulation which can be taken once a day and can be released stably and effectively for 24 hours. The specific solution is as follows:
1. A prolonged-release oral solid formulation of pirfenidone, comprising a sustained-release formulation, wherein the sustained-release formulation contains a swelling material, a shaping material and an adhesive material;
   the weight percentage of the swelling material in the prolonged-release oral solid formulation is 10%-25%; a weight percentage of the shaping material in the prolonged-release oral solid formulation is 10%-25%; and a weight percentage of the adhesive material in the prolonged-release oral solid formulation is 1%-8%;
   the viscosity of the swelling material is in a range of 500 mpa.s-300,000 mpa.s, the viscosity of the adhesive material is in a range of 50 mpa.s -100,000 mpa.s, and the viscosity of the shaping material is in a range of 50 mpa.s -60,000 mpa.s;
   the swelling material is selected from one or more of sodium carboxymethylcellulose, glyceryl behenate, sodium alginate, calcium alginate, potassium alginate, hydroxypropyl methylcellulose (HPMC), carbomer, ammonium alginate, carnauba wax, tragacanth gum, arabic gum, and shellac;
   the shaping material is selected from one or more of sodium carboxymethylcellulose, calcium carboxymethylcellulose, ethyl cellulose, carbomer, polylactic acid, a polylactic acid-glycolic acid copolymer, sodium alginate, hydrogenated vegetable oil, polymethacrylate, beeswax, glyceryl monostearate, polyethylene, and an ethylene-vinyl acetate copolymer;
   the adhesive material is selected from one or more of hydroxyethyl cellulose, gelatin, polyoxyethylene, carbomer, pectin, chitosan, glucose, alginate, fucosamine, starch, and chitin; and
   a blood drug concentration parameter achieved by the prolonged-release oral solid formulation when administered once a day to an individual at a daily dose is that: within 24 hours after an administration, a ratio of a maximum blood drug concentration (Cₘₐₓ) to a minimum blood drug concentration (Cₘᵢₙ) corresponding to a blood drug concentration curve is within 2-10, preferably within 2-5, and more preferably within 2-3.5.
2. The prolonged-release oral solid formulation of the pirfenidone according to claim 1, wherein a proportion of the sustained-release formulation to a total tablet weight is 50%-100%, or any value in the range; or the proportion of the sustained-release formulation to the total tablet weight is 50%, 60%, 65%, 70%, 73%, 78%, 80%, 85%, 90%, or a range formed by any two of these values.
3. The prolonged-release oral solid formulation of pirfenidone according to claim 2, wherein
   an active pharmaceutical ingredient (API) in the sustained-release formulation accounts for 50%-100% of a total API, or any value in the range; or the proportion of the API in the sustained-release formulation in the total API is 50%, 60%, 65%, 70%, 73%, 78%, 85%, 73%, 80%, 85%, 90%, or a range formed by any two of these values.
4. The prolonged-release oral solid formulation of the pirfenidone according to claim 1, wherein a ratio of the swelling material to the adhesive material is 1-9:1 or any value in the range, or 8:1, 7:1, 6:1, 5:1, 4:1, 3:1, 2:1 or 1:1; and
   a ratio of the adhesive material to the shaping material is (0.2-1) :1 or any value in the range, or 0.3:1, 0.4:1, 0.5:1, 0.6:1, 0.7:1, 0.8:1 or 0.9:1.
5. The prolonged-release oral solid formulation of the pirfenidone according to claim 2, wherein:
   the prolonged-release oral solid formulation is a single-layer tablet, and the sustained-release formulation accounts for 100% of the weight of the single-layer tablet.
6. The prolonged-release oral solid formulation of the pirfenidone according to claim 2, wherein
   the prolonged-release oral solid formulation further comprises an immediate-release formulation, and a ratio of the immediate-release formulation to the sustained-release formulation is 1-10, and preferably 2-8.
7. The prolonged-release oral solid formulation of the pirfenidone according to claim 6, wherein a ratio of an API in the immediate-release formulation to an API in the sustained-release formulation is 0.1-0.6.
8. The prolonged-release oral solid formulation of the pirfenidone according to claim 1, wherein
   the viscosity of the swelling material is selected from any of the following ranges: 500 mpa.s - 5,000 mpa.s, 500 mpa.s-1,000 mpa.s, 2,000 mpa.s -5,000 mpa.s, 50,000 mpa.s -150,000 mpa.s or 150,000 mpa.s -300,000 mpa.s;
   the viscosity of the shaping material is selected from any of the following ranges: 55 mpa.s - 60,000 mpa.s, 55 mpa.s -10,000 mpa.s, 55 mpa.s -1,000 mpa.s, or 60 mpa.s -200 mpa.s; and
   the viscosity of the adhesive material is selected from any of the following ranges: 55 mpa.s - 100,000 mpa.s, 60 mpa.s -200 mpa.s, 65 mpa.s -10,000 mpa.s, 4,000 mpa.s -10,000 mpa.s, 5,000 mpa.s -10,000 mpa.s, 5,500 mpa.s -7,500 mpa.s or 7,000 mpa.s -10,000 mpa.s.
9. The prolonged-release oral solid formulation of pirfenidone according to claim 1, wherein
   the swelling material employs the HPMC with a model selected from one or more of K100LV, K4M, K750, K100, K200M, and E5LV; and
   a molecular weight of the polyoxyethylene is 100,000-7,000,000.
10. The prolonged-release oral solid formulation of the pirfenidone according to any one of claims 1-9, wherein the prolonged-release oral solid formulation is a tablet, and minimum sizes of the tablet in three dimensions of a length, a width and a height are not less than 8.0 mm;
   the tablet with the minimum size in three dimension has an expansion growth rate satisfy the following conditions:
   in 900 mL of a medium of pH 4.5 at 37°C, and at a rotation speed of 75 rpm;
   the expansion growth rate within 1 h is ≥ 4.5%; the growth rate within 2 h is ≥ 12%; the expansion growth rate within 4 h is ≥ 25%; the growth rate within 6 h is ≥ 20%; and the expansion growth rate within 10 h is ≥ 40%.
11. The prolonged-release oral solid formulation of the pirfenidone according to claim 10, having any of the following specifications:
   (1) a content of the pirfenidone is 500 mg-700 mg;
   (2) the content of the pirfenidone is 600 mg;
   (3) the content of the pirfenidone is 700 mg-1,000 mg; or
   (4) the content of the pirfenidone is 801 mg.
12. The prolonged-release oral solid formulation of the pirfenidone according to any one of claims 1-9, wherein: according to a USP standard 2 method, in 900 mL of the medium of pH 4.5 at 37°C and at a rotation speed of 75 rpm, from the prolonged-release oral solid formulation more than 15% of an active substance is dissolved out within 0.5 h; more than 25% of the active substance is dissolved out within 2 h, 50-70% of the active substance is dissolved out within 10 h, and more than 80% of the active substance is dissolved out within 20 h.
13. The prolonged-release oral solid formulation of the pirfenidone according to claim 11, wherein when the prolonged-release oral solid formulation is used for the administration to an individual, the prolonged-release oral solid formulation has the following parameter characteristics: the prolonged-release oral solid formulation can stay in a stomach for at least 12 h; and within a single dosing interval of 24 h, a continuous time of a blood drug concentration being higher than 300 ng/mL is ≥ 15 h, and a Cₘₐₓ is not higher than 3,500 ng/mL, preferably the Cₘₐₓ is not higher than 2,500 ng/mL.
14. The prolonged-release oral solid formulation of pirfenidone according to claim 11, wherein when the prolonged-release oral solid formulation is used for administration to an individual, the blood drug concentration parameters comprise: the Cₘₐₓ of 800 ng/mL-3,500 ng/mL, and preferably 1,000ng/mL-2,500 ng/mL, and the Cₘᵢₙ ≥ 300 ng/mL and preferably the Cₘᵢₙ ≥ 400 ng/mL.
15. The prolonged-release oral solid formulation of pirfenidone according to claim 1, wherein the prolonged-release oral solid formulation is a tablet or a capsule, wherein the tablet is allowed to be a single-layer tablet, a double-layer tablet, a three-layer tablet, a core-coated tablet, or a ring-shaped tablet.
16. The prolonged-release oral solid formulation of the pirfenidone according to claim 1, wherein the prolonged-release oral solid formulation is a three-layer tablet, a middle layer is not less than 10% of a total weight of the tablet, and the third layer is not less than 15% of the total weight of the three-layer tablet.

In view of the problems that the existing oral products of pirfenidone have poor compliance caused by dosing for multiple times a day, can only be used after meals and have blood concentrations that fluctuate greatly,
the present invention develops a prolonged-release oral formulation which can be taken once a day before or after meals. After the formulation developed by the present invention enters the human body, a part of the drug is rapidly released to reach a peak value, and the other part of the drug is slowly released as controlled through an expanded tablet structure, thereby ensuring that the peak blood drug concentration Cmax will not drop suddenly, the drug is released steadily and effectively within 24 hours, the blood drug concentration is stable, and the peak-to-valley ratio can be close to 2 and lower than 3, thereby reducing adverse responses caused by the unstable blood drug concentration and the high peak-to-valley ratio.

### Term definition

Maximum blood concentration Cmax: the maximum blood concentration achieved by a drug entering the blood after administration.

Minimum blood concentration Cmin: the minimum blood concentration reached by the drug entering the blood after administration.

### BRIEF DESCRIPTION OF DRAWINGS

FIG. 1 shows a curve of an expansion growth rate in length of pirfenidone double-layer tablets of Example 1 and Comparative Examples 1, 2, and 3 of the present invention;
FIG. 2 shows a curve of an expansion growth rate in width of the pirfenidone double-layer tablets of Example 1 and Comparative Examples 1, 2, and 3 of the present invention;
FIG. 3 shows a curve of an expansion growth rate in height of the pirfenidone double-layer tablets of Example 1 and Comparative Examples 1, 2, and 3 of the present invention;
FIG. 4 shows a curve of an expansion growth rate in length of pirfenidone double-layer tablets of Example 2 and Comparative Examples 4, 5, and 6 of the present invention;
FIG. 5 shows a curve of an expansion growth rate in width of the pirfenidone double-layer tablets of Example 2 and Comparative Examples 4, 5, and 6 of the present invention;
FIG. 6 shows a curve of an expansion growth rate in height of the pirfenidone double-layer tablets of Example 2 and Comparative Examples 4, 5, and 6 of the present invention;
FIG. 7 shows a curve of an expansion growth rate in length of pirfenidone double-layer tablets of Example 3 and Comparative Examples 7 and 8 of the present invention;
FIG. 8 shows a curve of an expansion growth rate in width of the pirfenidone double-layer tablets of Example 3 and Comparative Examples 7 and 8 of the present invention;
FIG. 9 shows a curve of an expansion growth rate in height of the pirfenidone double-layer tablets of Example 3 and Comparative Examples 7 and 8 of the present invention;
FIG. 10 shows a curve of an expansion growth rate in length of pirfenidone double-layer tablets of Example 4 and Comparative Examples 9 and 10 of the present invention;
FIG. 11 shows a curve of an expansion growth rate in width of pirfenidone double-layer tablets of Example 5 and Comparative Examples 9 and 10 of the present invention;
FIG. 12 shows a curve of an expansion growth rate in height of pirfenidone double-layer tablets of Example 6 and Comparative Examples 9 and 10 of the present invention;
FIG. 13 shows a cumulative dissolution curve of Example 1 and Comparative Examples 1, 2, and 3;
FIG. 14 shows a cumulative dissolution curve of Example 2 and Comparative Examples 4, 5, and 6;
FIG. 15 shows a cumulative dissolution curve of Example 3 and Comparative Examples 7 and 8;
FIG. 16 shows a cumulative dissolution curve of Example 4 and Comparative Examples 9 and 10; and
FIG. 17 shows a comparative curve of blood drug concentration of the pirfenidone double-layer tablet taken orally once a day of the present invention relative to the existing pirfenidone tablet taken three times daily.

### DETAILED DESCRIPTION

### I. Prescriptions of Examples and Comparative Examples

### Example 1. Preparation of 1,000 Pirfenidone Double-layer Tablets

**Table 1. 1,000 pirfenidone double-layer tablets prepared according to components shown in the table below:**

| Layes of tablet | Material Name | Mass (g) | Function |
|---|---|---|---|
| Immediate-release layer | Pirfenidone | 150 | API |
| | Pregelatinized starch | 30 | Filler |
| | Microcrystalline cellulose PH101 | 60 | Filler |
| | Crospovidone | 8 | Disintegrant |
| | Sodium stearyl fumarate | 2 | Flow aid |
| | Pirfenidone | 450 | |
| | Sodium alginate | 160 | API |
| | HPMC K100M | 100 | Shaping system |
| | PVP K30 | 52 | Swelling system |
| Sustained-release layer | HPMC K200M | 200 | Adhesive |
| | PEO(N80) | 60 | Swelling system |
| | Microcrystalline cellulose PH101 | 53 | Adhesion system |
| | Talcum powder | 10 | Filler |
| | Colloidal Silica | 24 | Flow aid |

The preparation process of Example 1 specifically included the following steps:
A. preparation of drug-containing granules for the immediate-release layer: 150 g of pirfenidone, 30 g of pregelatinized starch, 60 g of microcrystalline cellulose, and 8 g of cross-linked polyvinylpyrrolidone were weighed, mixed evenly, granulated, subjected to wet granulation, and dried; and then added with 2 g of sodium stearyl fumarate, and mixed to total mixed granules of the immediate-release layer for later use;
B. preparation of drug-containing granules for the sustained-release layer: 450 g of pirfenidone, 160 g of sodium alginate, 100 g of HPMC K100M, 200 g of HPMC K200M, 60 g of PEO, and 53 g of microcrystalline cellulose were weighed, mixed for 3-5 min , added with 52 g of PVP K30 for making a soft material, subjected to wet granulation, and dried; then added with 10 g of talc powder and 24 g of colloidal silica, and mixed to obtain the total mixed granules of the sustained-release layer for use;
C. compression of double-layer tablets: an appropriate amount of the drug-containing granules for the sustained-release layer was weighed and placed into a die of a tablet press for pre-compression; then an appropriate amount of the drug-containing granules for the immediate-release layer was weighed and placed into the die of the tablet press for compression into double-layer tablets.

### Comparative Example 1. Preparation of 1,000 Pirfenidone Double-layer Tablets

**Table 2. 1,000 pirfenidone double-layer tablets prepared according to components shown in the table below:**

| Layes of tablet | Material Name | Mass (g) | Function |
|---|---|---|---|
| Immediate-release layer | Pirfenidone | 150 | API |
| | Pregelatinized starch | 30 | Filler |
| | Microcrystalline cellulose PH101 | 60 | Filler |
| | Crospovidone | 8 | Disintegrant |
| | Sodium stearyl fumarate | 2 | Flow aid |
| Sustained-release layer | Pirfenidone | 450 | |
| | Sodium alginate | 160 | API |
| | HPMC K100M | 200 | Shaping system |
| | PVP K30 | 52 | Swelling system |
| | HPMC K200M | 200 | Adhesive |
| | PEO(N80) | 60 | Swelling system |
| | Microcrystalline cellulose PH101 | 93 | Adhesion system |
| | Talcum powder | 10 | Filler |
| | Colloidal Silica | 24 | Flow aid |

The preparation process of Comparative Example 1 was consistent with that of Example 1, and double-layer tablets with the same shape and size were prepared.

The prescription of Comparative Example 1 was different from that of Example 1 in that, in the sustained-release layer in Comparative Example 1, the unit prescription dosage of HPMC K100M was 200 mg, and the unit prescription dosage of microcrystalline cellulose PH101 was 93 mg, and the other prescriptions were the same as those in Example 1.

A ratio of a swelling material to the total weight of the tablet in Comparative Example 1 was 26.68%, and the ratio of the swelling material to the total weight of the tablet in Example 1 was 22.08%.

### Comparative Example 2. Preparation of 1,000 Pirfenidone Double-layer Tablets

**Table 3. 1,000 pirfenidone double-layer tablets prepared according to components shown in the table below:**

| Layes of tablet | Material Name | Mass (g) | Function |
|---|---|---|---|
| Immediate-release layer | Pirfenidone | 150 | API |
| | Pregelatinized starch | 30 | Filler |
| | Microcrystalline cellulose PH101 | 60 | Filler |
| | Crospovidone | 8 | Disintegrant |
| | Sodium stearyl fumarate | 2 | Flow aid |
| Sustained-release layer | Pirfenidone | 450 | |
| | Sodium alginate | 100 | API |
| | HPMC K100M | 100 | Shaping system |
| | PVP K30 | 52 | Swelling system |
| | HPMC K200M | 200 | Adhesive |
| | PEO(N80) | 60 | Swelling system |
| | Microcrystalline cellulose PH101 | 153 | Adhesion system |
| | Talcum powder | 10 | Filler |
| | Colloidal Silica | 24 | Flow aid |

The preparation process of Comparative Example 2 was consistent with that of Example 1, and double-layer tablets with the same shape and size were prepared.

The prescription of Comparative Example 2 was different from that of Example 1 in that, in the sustained-release layer in Comparative Example 1, the unit prescription dosage of sodium alginate was 100 mg, and the unit prescription dosage of microcrystalline cellulose PH101 was 153 mg, and the remainder was the same as that in Example 1.

A ratio of a shaping material to the total weight of the tablet in Comparative Example 2 was 7.15%, and the ratio of the shaping material to the total weight of the tablet in Example 1 was 11.77%.

### Comparative Example 3. Preparation of 1,000 Pirfenidone Double-layer Tablets

**Table 4. 1,000 pirfenidone double-layer tablets prepared according to components shown in the table below:**

| Layes of tablet | Material Name | Mass (g) | Function |
|---|---|---|---|
| Immediate-release layer | Pirfenidone | 150 | API |
| | Pregelatinized starch | 30 | Filler |
| | Microcrystalline cellulose PH101 | 60 | Filler |
| | Crospovidone | 8 | Disintegrant |
| | Sodium stearyl fumarate | 2 | Flow aid |
| Sustained-release layer | Pirfenidone | 450 | |
| | Sodium alginate | 160 | API |
| | HPMC K100M | 100 | Shaping system |
| | PVP K30 | 52 | Swelling system |
| | HPMC K200M | 200 | Adhesive |
| | PEO(N80) | 150 | Swelling system |
| | Microcrystalline cellulose PH101 | 53 | Adhesion system |
| | Talcum powder | 10 | Filler |
| | Colloidal Silica | 24 | Flow aid |

The preparation process of Comparative Example 3 was consistent with that of Example 1, and double-layer tablets with the same shape and size were prepared.

The prescription of Comparative Example 3 was different from that of Example 1 in that, in the sustained-release layer in Comparative Example 1, the unit prescription dosage of PEO (N80) was 150 mg, and the remainder was the same as that in Example 1.

A ratio of an adhesive material to the total weight of the tablet in Comparative Example 3 was 10.35%, and the ratio of the adhesive material to the total weight of the tablet in Example 1 was 4.42%.

### Example 2. Preparation of 1,000 Pirfenidone Double-layer Tablets

**Table 5. 1,000 pirfenidone double-layer tablets prepared according to components shown in the table below:**

| Layes of tablet | Material Name | Mass (g) | Function |
|---|---|---|---|
| Immediate-release layer | Pirfenidone | 80 | API |
| | Pregelatinized starch | 50 | Filler |
| | Microcrystalline cellulose PH101 | 50 | Filler |
| | Crosslinked sodium carboxylmethyl cellulose | 18 | Disintegrant |
| | Magnesium stearate | 2 | Flow aid |
| Sustained-release layer | Pirfenidone | 520 | |
| | Sodium alginate | 280 | API |
| | HPMC K750 | 100 | Shaping system |
| | PVP K30 | 52 | Swelling system |
| | HPMC K100M | 200 | Adhesive |
| | PEO(N80) | 100 | Swelling system |
| | Microcrystalline cellulose PH101 | 53 | Adhesion system |
| | Talcum powder | 10 | Filler |
| | Colloidal Silica | 24 | Flow aid |

The preparation process of Example 2 was consistent with that of Example 1, and double-layer tablets with the same shape and size were prepared.

The specific preparation process was:
A. preparation of drug-containing granules for the immediate-release layer: 80 g of pirfenidone, 50 g of pregelatinized starch, 50 g of microcrystalline cellulose, and 8 g of crosslinked sodium carboxylmethyl cellulose were weighed, mixed evenly, granulated, subjected to wet granulation, and dried; then added with 2 g of magnesium stearate, and mixed to obtain the total mixed granules of the immediate-release layer for later use;
B. preparation of drug-containing granules for the sustained-release layer: 520 g of pirfenidone, 280 g of sodium alginate, 100 g of HPMC K750, 200 g of HPMC K100M, 100 g of PEO, and 53 g of microcrystalline cellulose were weighed, mixed for 3-5 min, added with 52 g of PVP K30 for making a soft material, subjected to wet granulation, and dried; then added with 10 g of talc powder and 24 g of colloidal silica, and mixed to obtain the total mixed granules of the sustained-release layer for later use;
C. compression of double-layer tablets: an appropriate amount of the drug-containing granules for the sustained-release layer was weighed and placed into a die of a tablet press for pre-compression; then an appropriate amount of the drug-containing granules for the immediate-release layer was weighed and placed into the die of the tablet press for compression into double-layer tablets.

### Comparative Example 4. Preparation of 1,000 Pirfenidone Double-layer Tablets

**Table 6. 1,000 pirfenidone double-layer tablets prepared according to components shown in the table below:**

| Layes of tablet | Material Name | Mass (g) | Function |
|---|---|---|---|
| Immediate-release layer | Pirfenidone | 80 | API |
| | Pregelatinized starch | 50 | Filler |
| | Microcrystalline cellulose PH101 | 50 | Filler |
| | Crosslinked sodium | 18 | Disintegrant |
| | carboxylmethyl cellulose | | |
| | Magnesium stearate | 2 | Flow aid |
| Sustained-release layer | Pirfenidone | 520 | |
| | Sodium alginate | 280 | API |
| | HPMC K100LV | 300 | Shaping system |
| | PVP K30 | 52 | Swelling system |
| | PEO(N80) | 100 | Adhesive |
| | Microcrystalline cellulose PH101 | 53 | Swelling system |
| | Talcum powder | 10 | Adhesion system |
| | Colloidal Silica | 24 | Filler |

The preparation process of Comparative Example 4 was consistent with that of Example 2, and double-layer tablets with the same shape and size were prepared.

The main difference between Comparative Example 4 and Example 2 was that the viscosity of the swelling material was inconsistent. The viscosity of the used HPMC K100LV in Comparative Example 4 was in a range of 80-120 mpa.s; while the viscosity of the used HPMC K750 was in a range of 562-1,050 mpa.s and the viscosity of the used HPMC K100M was in a range of 75,000-140,000 mpa.s in Example 2.

### Comparative Example 5. Preparation of 1,000 Pirfenidone Double-layer Tablets

**Table 7. 1,000 pirfenidone double-layer tablets prepared according to components shown in the table below:**

| Layes of tablet | Material Name | Mass (g) | Function |
|---|---|---|---|
| Immediate-release layer | Pirfenidone | 80 | API |
| | Pregelatinized starch | 50 | Filler |
| | Microcrystalline cellulose PH101 | 50 | Filler |
| | Crosslinked sodium | 18 | Disintegrant |
| | carboxylmethyl cellulose | | |
| | Magnesium stearate | 2 | Flow aid |
| | Pirfenidone | 520 | |
| | Polyvinyl alcohol | 280 | API |
| | HPMC K750 | 100 | Shaping system |
| | PVP K30 | 52 | Swelling system |
| Sustained-release layer | HPMC K100M | 200 | Adhesive |
| | PEO(N80) | 100 | Swelling system |
| | Microcrystalline cellulose PH101 | 53 | Adhesion system |
| | Talcum powder | 10 | Filler |
| | Colloidal Silica | 24 | Flow aid |

The preparation process of Comparative Example 5 was consistent with that of Example 2, and double-layer tablets with the same shape and size were prepared.

The main difference between Comparative Example 5 and Example 2 was that the viscosity of the shaping material was inconsistent.

The viscosity of the sodium alginate used in Example 2 was in a range of 60-170 mPa.s; while the viscosity of the polyvinyl alcohol used in Comparative Example 5 was in a range of 20-30 mPa.s.

### Comparative Example 6. Preparation of 1,000 Pirfenidone Double-layer Tablets

**Table 8. 1,000 pirfenidone double-layer tablets prepared according to components shown in the table below:**

| Layes of tablet | Material Name | Mass (g) | Function |
|---|---|---|---|
| Immediate-release layer | Pirfenidone | 80 | API |
| | Pregelatinized starch | 50 | Filler |
| | Microcrystalline cellulose PH101 | 50 | Filler |
| | Crosslinked sodium | 18 | Disintegrant |
| | carboxylmethyl cellulose | | |
| | Magnesium stearate | 2 | Flow aid |
| | Pirfenidone | 520 | |
| | Sodium alginate | 280 | API |
| | HPMC K750 | 100 | Shaping system |
| | PVP K30 | 52 | Swelling system |
| Sustained-release layer | HPMC K100M | 200 | Adhesive |
| | Chitosan | 100 | Swelling system |
| | Microcrystalline cellulose PH101 | 53 | Adhesion system |
| | Talcum powder | 10 | Filler |
| | Colloidal Silica | 24 | Flow aid |

The preparation process of Comparative Example 6 was consistent with that of Example 2, and double-layer tablets with the same shape and size were prepared.

The main difference between Comparative Example 6 and Example 2 was that the viscosity of the adhesive material was inconsistent. The viscosity of the chitosan used in Comparative Example 6 was in a range of 1-40 mPa.s; while the viscosity of the PEO (N80) used in Example 2 was in a range of 65-90 mPa.s.

### Example 3. Preparation of 1,000 Pirfenidone Double-layer Tablets

**Table 9. 1,000 pirfenidone double-layer tablets prepared according to components shown in the table below:**

| Layes of tablet | Material Name | Mass (g) | Function |
|---|---|---|---|
| Immediate-release layer | Pirfenidone | 220 | API |
| | Pregelatinized starch | 30 | Filler |
| | Microcrystalline cellulose PH101 | 60 | Filler |
| | Crosslinked sodium carboxylmethyl cellulose | 8 | Disintegrant |
| | Magnesium stearate | 2 | Flow aid |
| Sustained-release layer | Pirfenidone | 380 | |
| | Sodium alginate | 190 | API |
| | HPMC K100M | 250 | Shaping system |
| | PVP K30 | 52 | Swelling system |
| | PEO(303) | 100 | Adhesive |
| | Microcrystalline cellulose PH101 | 53 | Swelling system |
| | Magnesium stearate | 10 | Adhesion system |
| | Colloidal Silica | 24 | Filler |

The preparation process of Example 3 was consistent with that of Example 1, and double-layer tablets with the same shape and size were prepared.

### Comparative Example 7. Preparation of 1,000 Pirfenidone Double-layer Tablets

**Table 10. 1,000 pirfenidone double-layer tablets prepared according to components shown in the table below:**

| Layes of tablet | Material Name | Mass (g) | Function |
|---|---|---|---|
| Immediate-release layer | Pirfenidone | 220 | API |
| | Pregelatinized starch | 300 | Filler |
| | Microcrystalline cellulose PH101 | 350 | Filler |
| | Crosslinked sodium carboxylmethyl cellulose | 48 | Disintegrant |
| | Magnesium stearate | 2 | Flow aid |
| Sustained-release layer | Pirfenidone | 380 | |
| | Sodium alginate | 190 | API |
| | HPMC K100M | 200 | Shaping system |
| | PVP K30 | 52 | Swelling system |
| | PEO(303) | 50 | Adhesive |
| | Microcrystalline cellulose PH101 | 53 | Swelling system |
| | Magnesium stearate | 10 | Adhesion system |
| | Colloidal Silica | 24 | Filler |

The preparation process of Comparative Example 7 was consistent with that of Example 3, and double-layer tablets with the same shape and size were prepared.

The main difference between Comparative Example 7 and Example 3 was that a ratio of the sustained-release layer to the total weight of the tablet in Comparative Example 7 was 49.6%, and the ratio of the sustained-release layer to the total weight of the tablet in Example 3 was 76.8%.

### Comparative Example 8. Preparation of 1,000 Pirfenidone Double-layer Tablets

**Table 11. 1,000 pirfenidone double-layer tablets prepared according to components shown in the table below:**

| Layes of tablet | Material Name | Mass (g) | Function |
|---|---|---|---|
| Immediate-release layer | Pirfenidone | 50 | API |
| | Pregelatinized starch | 30 | Filler |
| | Microcrystalline cellulose PH101 | 60 | Filler |
| | Crosslinked sodium carboxylmethyl cellulose | 8 | Disintegrant |
| | Magnesium stearate | 2 | Flow aid |
| Sustained-release layer | Pirfenidone | 550 | |
| | Sodium alginate | 190 | API |
| | HPMC K100M | 250 | Shaping system |
| | PVP K30 | 52 | Swelling system |
| | PEO(303) | 100 | Adhesive |
| | Microcrystalline cellulose PH101 | 53 | Swelling system |
| | Magnesium stearate | 10 | Adhesion system |
| | Colloidal Silica | 24 | Filler |

The preparation process of Comparative Example 8 was consistent with that of Example 3, and double-layer tablets with the same shape and size were prepared.

The main difference between Comparative Example 8 and Example 3 was that, in Comparative Example 8, the unit prescription dosage of pirfenidone in the immediate-release layer of was 50 mg, and the unit prescription dosage of pirfenidone in the sustained-release layer was 550 mg; while in Example 3, the unit prescription dosage of pirfenidone in the immediate-release layer was 220 mg, and the unit prescription dosage of pirfenidone in the sustained-release layer was 380 mg.

### Example 4. Preparation of 1,000 Pirfenidone Double-layer Tablets

**Table 12. 1,000 pirfenidone double-layer tablets prepared according to components shown in the table below:**

| Layes of tablet | Material Name | Mass (g) | Function |
|---|---|---|---|
| Immediate-release layer | Pirfenidone | 100 | API |
| | Pregelatinized starch | 30 | Filler |
| | Microcrystalline cellulose PH101 | 60 | Filler |
| | Crosslinked sodium carboxylmethyl cellulose | 8 | Disintegrant |
| | Magnesium stearate | 2 | Flow aid |
| Sustained-release layer | Pirfenidone | 500 | |
| | Sodium alginate | 210 | API |
| | HPMC K100M | 250 | Shaping system |
| | PVP K30 | 52 | Swelling system |
| | PEO(303) | 100 | Adhesive |
| | Microcrystalline cellulose PH101 | 53 | Swelling system |
| | Magnesium stearate | 10 | Adhesion system |
| | Colloidal Silica | 24 | Filler |

The preparation process of Example 4 was consistent with that of Example 1, and double-layer tablets with the same shape and size were prepared.

The specific preparation process was:
A. preparation of drug-containing granules for the immediate-release layer: 100 g of pirfenidone, 30 g of pregelatinized starch, 60 g of microcrystalline cellulose, and 8 g of crosslinked sodium carboxylmethyl cellulose were weighed, mixed evenly, granulated, subjected to wet granulation, and dried; then added with 2 g of magnesium stearate, and mixed to obtain the total mixed granules of the immediate-release layer for later use;
B. preparation of drug-containing granules for the sustained-release layer: 500 g of pirfenidone, 210 g of sodium alginate, 250 g of HPMC K100M, 100 g of PEO, and 52 g of microcrystalline cellulose were weighed, mixed for 3-5 min, added with 52 g of PVP K30 for making a soft material, subjected to wet granulation, and dried; then added with 10 g of magnesium stearate and 24 g of colloidal silicon dioxide, and mixed to obtain the total mixed granules of the sustained-release layer for later use;
C. compression of double-layer tablets: an appropriate amount of the drug-containing granules for the sustained-release layer was weighed and placed into a die of a tablet press for pre-compression; then an appropriate amount of the drug-containing granules for the immediate-release layer was weighed and placed into the die of the tablet press for compression into double-layer tablets.

### Comparative Example 9. Preparation of 1,000 Pirfenidone Double-layer Tablets

**Table 13. 1,000 pirfenidone double-layer tablets prepared according to components shown in the table below:**

| Layes of tablet | Material Name | Mass (g) | Function |
|---|---|---|---|
| Immediate-release layer | Pirfenidone | 100 | API |
| | Pregelatinized starch | 30 | Filler |
| | Microcrystalline cellulose PH101 | 60 | Filler |
| | Crosslinked sodium carboxylmethyl cellulose | 8 | Disintegrant |
| | Magnesium stearate | 2 | Flow aid |
| Sustained-release layer | Pirfenidone | 500 | |
| | Sodium alginate | 210 | API |
| | HPMC K100M | 500 | Shaping system |
| | PVP K30 | 52 | Swelling system |
| | PEO(303) | 50 | Adhesive |
| | Microcrystalline cellulose PH101 | 53 | Swelling system |
| | Magnesium stearate | 10 | Adhesion system |
| | Colloidal Silica | 24 | Filler |

The preparation process of Comparative Example 9 was consistent with that of Example 4, and double-layer tablets with the same shape and size were prepared.

The main difference between Comparative Example 9 and Example 4 was that, in the sustained-release layer of Comparative Example 9, the unit prescription dosage of the HPMC
K100M was 500 mg, the unit prescription dosage of the PEO (303) was 50 mg, and the swelling material:adhesive material = 10: 1;
while in the sustained-release layer of Example 4, the unit prescription dosage of the HPMC/K100M was 250 mg, the unit prescription dosage of the PEO (303) was100 mg, and the swelling material:adhesive material = 5:2.

### Comparative Example 10. Preparation of 1,000 Pirfenidone Double-layer Tablets

**Table 14. 1,000 pirfenidone double-layer tablets prepared according to components shown in the table below:**

| Layes of tablet | Material Name | Mass (g) | Function |
|---|---|---|---|
| Immediate-release layer | Pirfenidone | 100 | API |
| | Pregelatinized starch | 30 | Filler |
| | Microcrystalline cellulose PH101 | 60 | Filler |
| | Crosslinked sodium carboxylmethyl cellulose | 8 | Disintegrant |
| | Magnesium stearate | 2 | Flow aid |
| Sustained-release layer | Pirfenidone | 500 | |
| | Sodium alginate | 400 | API |
| | HPMC K100M | 250 | Shaping system |
| | PVP K30 | 52 | Swelling system |
| | PEO(303) | 70 | Adhesive |
| | Microcrystalline cellulose PH101 | 53 | Swelling system |
| | Magnesium stearate | 10 | Adhesion system |
| | Colloidal Silica | 24 | Filler |

The preparation process of Comparative Example 10 was consistent with that of Example 4, and double-layer tablets with the same shape and size were prepared.

The main difference between Comparative Example 10 and Example 4 was that, in the sustained-release layer of Comparative Example 10, the unit prescription dosage of sodium alginate was 400 mg, the unit prescription dosage of PEO (303) was 70 mg, and the adhesive material:shaping material = 7:40; while in the sustained-release layer of Example 4, the unit prescription dosage of the HPMCK100M was 210 mg, the unit prescription dosage of the PEO (303) was 100 mg, and the adhesive material: shaping material = 10:21.

### II. In vitro testing of formulations

**In order to ensure rapid onset of action (partial rapid release) and prolonged action within 24 hours after once-daily administration, the formulation must had an expansion growth rate to ensure that it stayed in the stomach long enough and that there was steady drug release during this period. Therefore, it was necessary to meet the standards in both the expansion growth rate test and the in vitro dissolution test.**

### 2.1 In vitro expansion study

Experiment conditions of in vitro dissolution: medium: pH 4.5, paddle method + sinker, 37°C, rotation speed: 75 rpm, and medium volume: 900 mL.

According to multiple small-scale experiments of the present invention, it was found that for tablets of 600-800 mg, the tablets prepared according to the formula of the present invention had a minimum size of not less than 8.0 mm in three dimensions of length, width and height, and the expansion growth rate of the dimension with the minimum size only needed to meet the following standards to ensure that the formulation stayed in the stomach for at least 12 hours through expansion, thereby achieving slow and prolonged release of API:
a growth rate within 1 h was ≥ 4.5%; the growth rate within 2 h was ≥ 12%; the growth rate within 4 h was ≥ 25%; the growth rate within 6 h was ≥ 20%; and the growth rate within 10 h was ≥ 40%.

The minimum three-dimensional size of the tablets in the examples of the present invention were: a length: 18.0-19.0 mm, a width: 10-11 mm, and a height: 8.0-9.5 mm. it had been found through experiments that the in vitro expansion growth rate of the tablets with the minimum size must meet the standards listed in Table 15 to ensure that the formulation stayed in the stomach for at least 12 hours through expansion to achieve slow and prolonged release of API.

**Table 15. In vitro expansion growth rate standards**

| Time h | Length growth rate % | Width growth rate % | Height growth rate % |
|---|---|---|---|
| 1 | ≥8 | ≥10 | ≥4.5 |
| 2 | ≥10 | ≥12 | ≥12 |
| 4 | ≥15 | ≥15 | ≥25 |
| 6 | ≥20 | ≥20 | ≥35 |
| 10 | ≥20 | ≥25 | ≥40 |

**Table 16. In vitro expansion growth rates of Example 1 and Comparative Examples 1, 2, and 3**

| | In vitro expansion growth rate (%) | | | |
|---|---|---|---|---|
| | Example 1 | Comparative Example 1 | Comparative Example 2 | Comparative Example 3 |
| Length - 1 h | 9.50 | 9.00 | 8.90 | 8.40 |
| Width - 1 h | 13.50 | 12.50 | 12.50 | 11.20 |
| Height - 1 h | 5.80 | 4.80 | 4.90 | 4.90 |
| Length - 2 h | 12.18 | 12.60 | 10.50 | 11.10 |
| Width - 2 h | 14.50 | 13.20 | ***11.40*** | ***11.20*** |
| Height - 2 h | 13.90 | ***11.80*** | ***11.80*** | ***11.90*** |
| Length - 4 h | 18.80 | 16.40 | 15.10 | 15.40 |
| Width - 4 h | 20.10 | 17.50 | 16.20 | 16.30 |
| Height - 4 h | 25.60 | ***23.80*** | ***21.20*** | ***21.30*** |
| Length - 6 h | 20.50 | ***19.00*** | ***17.80*** | ***17.90*** |
| Width - 6 h | 22.90 | 20.80 | ***18.40*** | ***18.30*** |
| Height - 6 h | 38.50 | ***33.20*** | ***28.50*** | ***28.60*** |
| Length - 10 h | 24.00 | 21.50 | ***19.50*** | ***19.70*** |
| Width - 10 h | 27.10 | ***24.80*** | ***21.20*** | ***21.90*** |
| Height - 10 h | 49.30 | 43.10 | ***38.70*** | ***39.10*** |

| | | | | |
|---|---|---|---|---|
| Note: the data in bold and italic were not in compliance with the standards. | | | | |

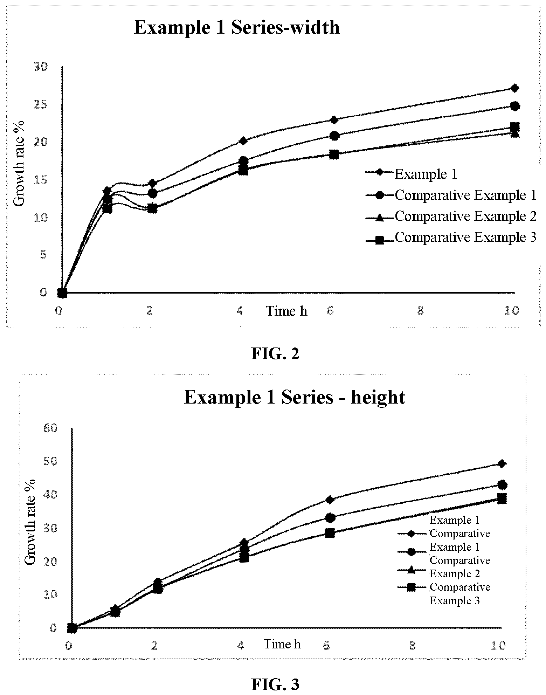

The curves of expansion growth rates of the pirfenidone double-layer tablets in Example 1 and Comparative Examples 1, 2, and 3 in terms of length, width, and height were shown in FIGs. 1-3.

It could be seen from Table 16 and FIGs. 1-3 that, when the proportion of the swelling material was higher than 25% (Comparative Example 1), the proportion of the shaping material was lower than 10 % (Comparative Example 2), and the proportion of the adhesive material (Comparative Example 3) was higher than 8%, starting from the 2nd hour, the expansion growth rate in at least one of the three dimensions of length, width and height of the tablet was unqualified, which would lead to the failure of relying on tablet expansion to control the slow and prolonged release of API.

**Table 17. In vitro expansion growth rates of Example 2 and Comparative Examples 4, 5, and 6**

| | In vitro expansion growth rate (%) | | | |
|---|---|---|---|---|
| | Example 2 | Comparative Example 4 | Comparative Example 5 | Comparative Example 6 |
| Length - 1 h | 10.00 | ***7.80*** | ***7.60*** | 8.10 |
| Width - 1 h | 13.60 | ***9.50*** | ***9.40*** | 10.50 |
| Height - 1 h | 6.80 | ***4.20*** | ***4.10*** | ***4.30*** |
| Length - 2 h | 13.10 | ***9.80*** | ***9.50*** | 10.20 |
| Width - 2 h | 14.90 | ***10.50*** | ***10.30*** | ***11.30*** |
| Height - 2 h | 13.80 | ***10.90*** | ***10.10*** | ***11.50*** |
| Length - 4 h | 19.50 | ***14.30*** | ***13.80*** | 15.40 |
| Width - 4 h | 20.10 | 15.6 | ***14.90*** | 16.20 |
| Height - 4 h | 26.80 | ***20.10*** | ***18.80*** | ***21.40*** |
| Length - 6 h | 21.60 | ***16.80*** | ***15.40*** | ***17.30*** |
| Width - 6 h | 23.40 | ***18.20*** | ***17.60*** | ***19.50*** |
| Height - 6 h | 37.20 | ***28.30*** | ***27.10*** | ***30.10*** |
| Length - 10 h | 24.00 | ***18.90*** | ***18.00*** | 20.00 |
| Width - 10 h | 27.80 | ***21.50*** | ***20.20*** | ***23.20*** |
| Height - 10 h | 50.10 | ***33.80*** | ***32.30*** | ***35.50*** |

| | | | | |
|---|---|---|---|---|
| Note: the data in bold and italic were not in compliance with the standards. | | | | |

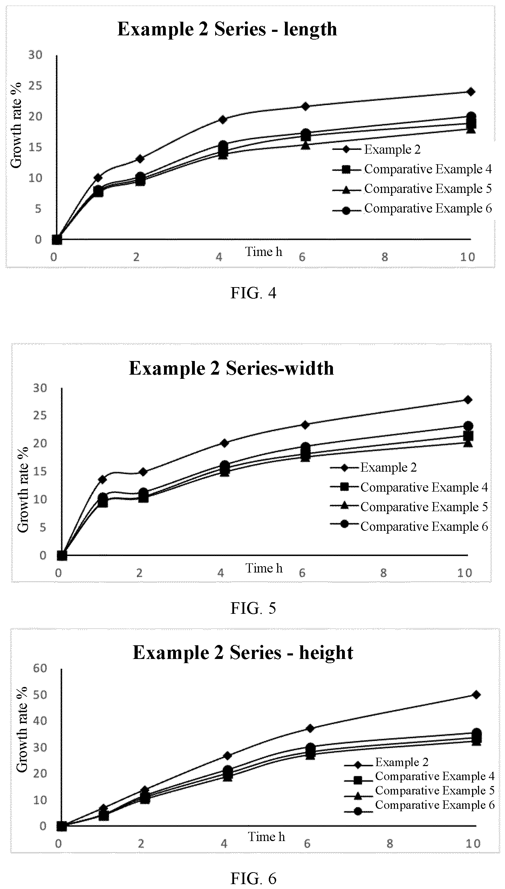

The curves of expansion growth rates of the pirfenidone double-layer tablets in Example 2 and Comparative Examples 4, 5, and 6 in terms of length, width, and height were shown in FIGs. 4-6.

It could be seen from Table 17 and FIGs. 4-6 that even if the ratios of the swelling material, the shaping material, and the adhesive material were within an appropriate range, when in the prescription the viscosity of the swelling material was lower than 500 mpa.s (Comparative Example 4), the viscosity of the shaping material was lower than 50 mpa.s (Comparative Example 5), and the viscosity of the adhesive material was lower than 50 mpa.s (Comparative Example 6), from the very beginning, the expansion growth rate in at least one of the three dimensions of length, width, and height of the tablet, especially in the high dimension with the minimum size was significantly unqualified, which would lead to the failure of relying on tablet expansion to control the slow and prolonged release of API.

**Table 18. In vitro expansion growth rates of Example 3 and Comparative Examples 7 and**

| | In vitro expansion growth rate (%) | |
|---|---|---|
| | Example 3 | Comparative Example 7 |
| Length - 1 h | 11.20 | ***7.50*** |
| Width - 1 h | 14.50 | ***8.90*** |
| Height - 1 h | 7.40 | ***4.00*** |
| Length - 2 h | 14.20 | ***9.20*** |
| Width - 2 h | 15.30 | ***10.10*** |
| Height - 2 h | 14.20 | ***10.20*** |
| Length - 4 h | 20.60 | ***13.10*** |
| Width - 4 h | 21.40 | ***14.50*** |
| Height - 4 h | 27.90 | ***18.20*** |
| Length - 6 h | 22.80 | ***14.90*** |
| Width - 6 h | 24.60 | ***16.50*** |
| Height - 6 h | 38.50 | ***26.30*** |
| Length - 10 h | 25.60 | ***17.80*** |
| Width - 10 h | 29.20 | ***19.50*** |
| Height - 10 h | 51.10 | ***31.50*** |

| | | |
|---|---|---|
| Note: the data in bold and italic were not in compliance with the standards. | | |

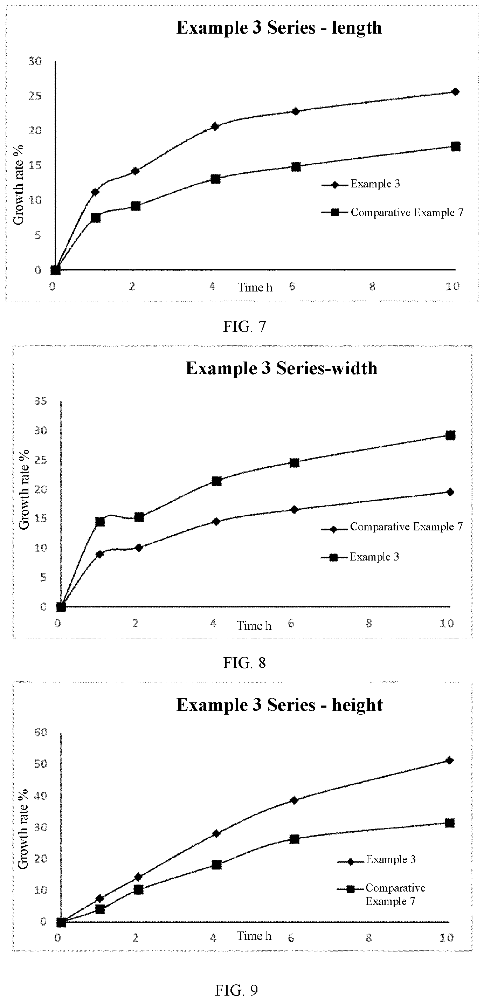

The curves of expansion growth rates of the pirfenidone double-layer tablets in Example 3 and Comparative Examples 7 and 8 in terms of length, width, and height were shown in FIGs. 7-9.

It could be seen from Table 18 and FIGs. 7, 8 and 9 that, when the ratio of the sustained-release layer to the total weight of the tablet was lower than 50% (Comparative Example 7), which would affect the expansion growth rate of the tablet.

The swelling, shaping and adhesive materials in the tablet were all in the sustained-release layer. The sustained-release layer must reach a certain ratio in the total weight of the tablet to ensure that the weight of the tablet contained enough swelling, the shaping and adhesion materials were employed to ensure the expansion rate and the thickness of the sustained-release layer, thereby ensuring that the tablet expanded to a certain sufficient volume in the stomach, delaying entry into the intestine and ensuring a smooth sustained-release.

**Table 19. In vitro expansion growth rates of Example 4 and Comparative Examples 9 and 10**

| | In vitro expansion growth rate (%) | | |
|---|---|---|---|
| | Example 4 | Comparative Example 9 | Comparative Example 10 |
| Length - 1 h | 11.30 | 12.40 | 10.50 |
| Width - 1 h | 14.70 | 15.60 | 13.80 |
| Height - 1 h | 8.10 | 10.00 | 7.90 |
| Length - 2 h | 14.80 | 16.50 | 13.50 |
| Width - 2 h | 15.90 | 17.50 | 14.20 |
| Height - 2 h | 15.10 | 18.90 | 14.80 |
| Length - 4 h | 21.30 | 24.50 | 20.60 |
| Width - 4 h | 22.60 | 25.60 | 21.30 |
| Height - 4 h | 28.80 | 35.30 | 27.40 |
| Length - 6 h | 23.84 | 29.80 | 22.60 |
| Width - 6 h | 25.60 | 30.50 | 24.50 |
| Height - 6 h | 39.80 | 45.80 | 38.50 |
| Length - 10 h | 27.30 | 35.60 | 26.60 |
| Width - 10 h | 30.10 | 40.10 | 29.10 |
| Height - 10 h | 53.40 | 60.20 | 52.60 |

| | | | |
|---|---|---|---|
| Note: the data in bold and italic were not in compliance with the standards. | | | |

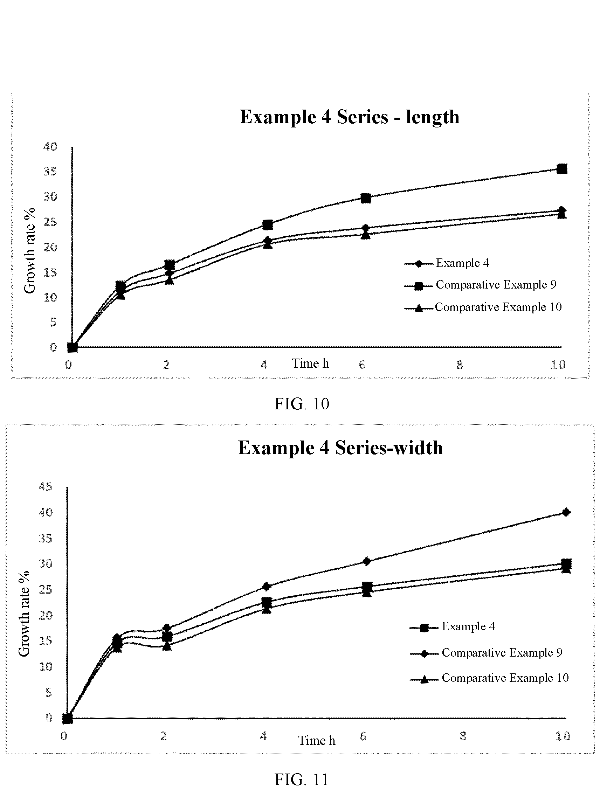

Curves of in vitro expansion growth rates of **pirfenidone double-layer tablets in length, width and height of Example 4 and Comparative Examples 9 and 10 were shown in** **FIGs. 10-12****.**

It could be seen from Table 19 and FIGs. 10-12 that, the fluctuation in the ratio of the swelling material:the adhesive material fluctuated (Comparative Example 9) or in the ratio of the adhesive material:the shaping material (Comparative Example 9) basically did not affect the expansion growth rate.

### 2.2 In vitro dissolution study

**Experiment conditions of in vitro dissolution:** medium: pH 4.5, paddle method + sinker, 37°C, rotation speed: 75 rpm, and medium volume: 900 mL.

After multiple small-scale experiments, it was determined that the in vitro dissolution must meet the standards in Table 20 to ensure that the blood drug concentration quickly reached the peak value when used in vivo and the drug was completely released within 24 hours and that the blood drug concentration remained within the effective concentration range, with a peak-to-valley ratio of no more than 3.

**Table 20. In vitro dissolution standards**

| Time (h) | Cumulative dissolution (%) |
|---|---|
| 0.5 | ≥15 |
| 2 | ≥25 |
| 10 | 50~70 |
| 20 | ≥80 |

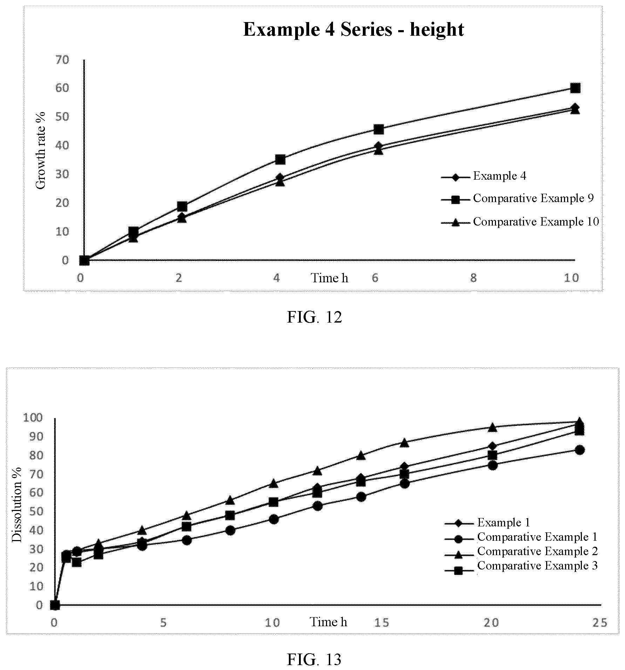

**Table 21 Cumulative dissolution of Example 1 and Comparative Examples 1, 2 and 3 as shown in table below and FIG. 13**

| | Cumulative dissolution (%) | | | |
|---|---|---|---|---|
| Time (h) | Example 1 | Comparative Example 1 | Comparative Example 2 | Comparative Example 3 |
| 0 | 0 | 0 | 0 | 0 |
| 0.5 | 26 | 27 | 25 | 26 |
| 1 | 28 | 29 | 29 | 23 |
| 2 | 30 | 30 | 33 | 27 |
| 4 | 34 | 32 | 40 | 33 |
| 6 | 42 | 35 | 48 | 42 |
| 8 | 48 | 40 | 56 | 48 |
| 10 | 55 | 46 | 65 | 55 |
| 12 | 63 | 53 | 72 | 60 |
| 14 | 68 | 58 | 80 | 66 |
| 16 | 74 | 65 | 87 | 70 |
| 20 | 85 | 75 | 95 | 80 |
| 24 | 97 | 83 | 98 | 93 |

It could be seen from Table 21 and FIG. 13 that when the ratio of the swelling material was higher than 25% (Comparative Example 1), the ratio of the shaping material was lower than 10% (Comparative Example 2), and the ratio of the adhesive material (Comparative Example 3) was higher than 8%, the ratio of the swelling material had a slight effect on the dissolution characteristics, and the dissolution characteristics were insensitive to the changes in the ratios of the shaping material and the adhesive material. In general, the swelling material, the shaping material and the adhesive material mainly affected the expansion characteristics.

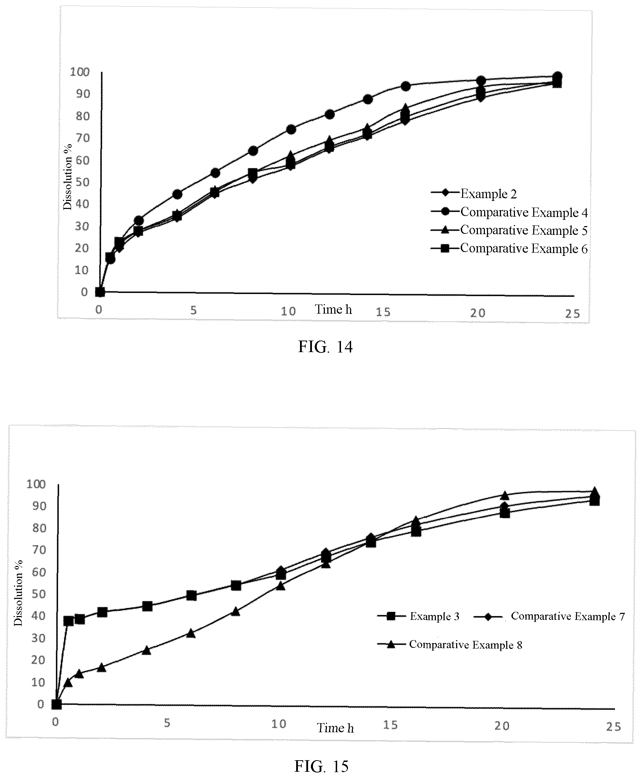

**Table 22. Cumulative dissolution of Example 2, and Comparative Examples 4, 5 and 6 as shown in table below and FIG. 14.**

| | Cumulative dissolution (%) | | | |
|---|---|---|---|---|
| Time (h) | Example 2 | Comparative Example 4 | Comparative Example 5 | Comparative Example 6 |
| 0 | 0 | 0 | 0 | 0 |
| 0.5 | 16 | 16 | 16 | 16 |
| 1 | 20 | 23 | 22 | 23 |
| 2 | 27 | 33 | 28 | 28 |
| 4 | 34 | 45 | 36 | 35 |
| 6 | 45 | 55 | 47 | 46 |
| 8 | 52 | 65 | 55 | 55 |
| 10 | 58 | 75 | 63 | 59 |
| 12 | 66 | 82 | 70 | 67 |
| 14 | 72 | 89 | 76 | 73 |
| 16 | 79 | 95 | 85 | 81 |
| 20 | 90 | 98 | 95 | 92 |
| 24 | 97 | 100 | 97 | 98 |

It could be seen from Table 22 and FIG. 14 that the viscosity of the swelling material (Comparative Example 4), the viscosity of the shaping material (Comparative Example 5), and the viscosity of the adhesive material (Comparative Example 6) had no significant effect on the dissolution of the tablets. It mainly affected the expansion characteristics.

**Table 23 Cumulative dissolution of Example 3 and Comparative Examples 7 and 8 as shown in table below and FIG. 15**

| | Cumulative dissolution (%) | | |
|---|---|---|---|
| Time (h) | Example 3 | Comparative Example 7 | Comparative Example 8 |
| 0 | 0 | 0 | 0 |
| 0.5 | 38 | 38 | 10 |
| 1 | 39 | 39 | 14 |
| 2 | 42 | 42 | 17 |
| 4 | 45 | 45 | 25 |
| 6 | 50 | 50 | 33 |
| 8 | 55 | 55 | 43 |
| 10 | 60 | 62 | 55 |
| 12 | 68 | 70 | 65 |
| 14 | 75 | 77 | 75 |
| 16 | 80 | 83 | 85 |
| 20 | 89 | 92 | 97 |
| 24 | 95 | 97 | 99 |

It could be seen from Table 23 and FIG. 15 that the fluctuation in the ratio of the sustained-release layer to the total weight of the tablet (Comparative Example 7) had no obvious effect on the dissolution characteristics. However, when the ratio of API in the immediate-release layer and the sustained-release layer was too low (Comparative Example 8), the early dissolution rate did not meet the standard. It could be inferred that the blood drug concentration could not quickly reach the peak value, affecting the efficacy of the drug.

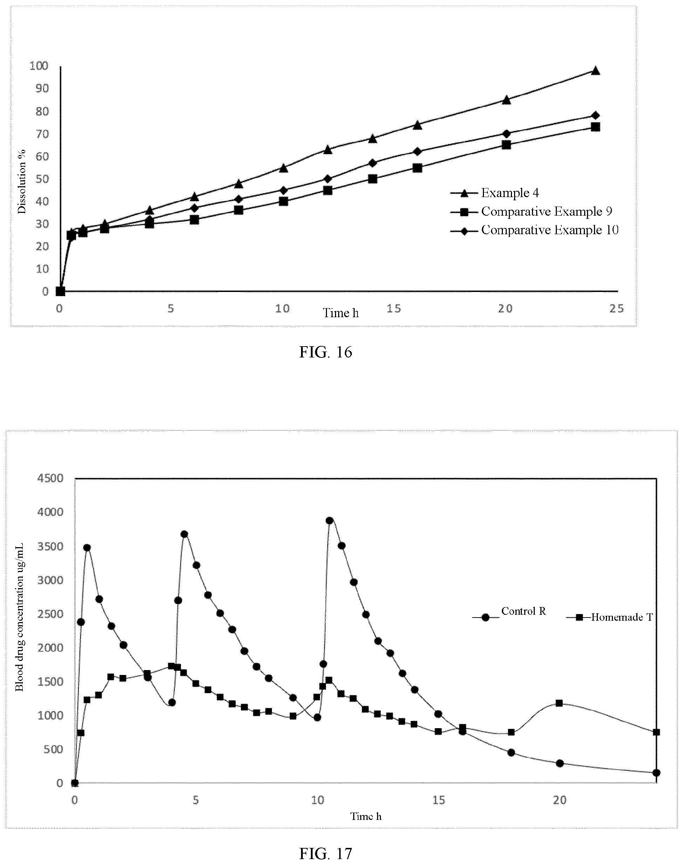

**Table 24 Cumulative dissolution of Example 4 and Comparative Examples 9 and 10 as shown in table below and FIG. 16**

| | Cumulative dissolution (%) | | |
|---|---|---|---|
| Time (h) | Example 4 | Comparative Example 9 | Comparative Example 10 |
| 0 | 0 | 0 | 0 |
| 0.5 | 26 | 25 | 24 |
| 1 | 28 | 26 | 26 |
| 2 | 30 | 28 | 28 |
| 4 | 36 | 30 | 32 |
| 6 | 42 | 32 | 37 |
| 8 | 48 | 36 | 41 |
| 10 | 55 | ***40*** | ***45*** |
| 12 | 63 | ***45*** | ***50*** |
| 14 | 68 | ***50*** | ***57*** |
| 16 | 74 | ***55*** | ***62*** |
| 20 | 85 | ***65*** | ***70*** |
| 24 | 98 | ***73*** | ***78*** |

Table 24 and FIG. 16 showed that when the ratio of the swelling material:the adhesive material was higher than 9 (Comparative Example 9) or when the ratio of the adhesive material:the shaping material was lower than 0.2 (Comparative Example 9), the dissolution did not meet the standard after the 10th hour, and the effect of the drug cannot be stably and effectively exerted for 24 hours.

### III. Comparison of in vivo blood drug concentration

Experiment: pirfenidone double-layer tablets prepared in Example 1 of the present invention, with each tablet of 600 mg, and 1 tablet every 24 hours;
Comparison: pirfenidone tablets, 200 mg, taken in the morning, noon and evening, 3 times a day, one tablet each time;
Experimental subjects: healthy subjects of 19-45 years old;
The results of blood drug concentration detection within 24 hours were shown in FIG. 17. The double-layer tablet of the present invention reached a peak value of 1,500-1,750 ng/mL about 1 hour after administration, and in the following 23 hours, the blood drug concentration was mostly maintained at a level of 750-1,500 ng/mL, with a peak-to-valley ratio of 2.33.

The control tablets were taken three times, producing three peaks in the range of 3,500-3,800 ng/mL, but they dropped to 1,000 ng/mL or below within 2-6 hours after administration. The lowest blood drug concentration was only about 200 ng/mL at 24 hours, and the peak-to-valley ratio reached 19:1.

It is further proved that the pirfenidone double-layer tablet developed by the present invention can quickly reach the peak blood drug concentration when taken once a day, and the blood drug concentration is stable within 24 hours, thereby achieving a 24-hour stable and effective effect. It does not need to be taken after meals, thereby effectively reducing the side effects caused by large fluctuations in blood drug concentration and improving drug compliance.

## Claims

1. A prolonged-release oral solid formulation of pirfenidone, comprising a sustained-release formulation, wherein the sustained-release formulation contains a swelling material, a shaping material and an adhesive material;
the weight percentage of the swelling material in the prolonged-release oral solid formulation is 10%-25%; a weight percentage of the shaping material in the prolonged-release oral solid formulation is 10%-25%; and a weight percentage of the adhesive material in the prolonged-release oral solid formulation is 1%-8%;
the viscosity of the swelling material is in a range of 500 mpa.s-300,000 mpa.s, the viscosity of the adhesive material is in a range of 50 mpa.s -100,000 mpa.s, and the viscosity of the shaping material is in a range of 50 mpa.s -60,000 mpa.s;
the swelling material is selected from one or more of sodium carboxymethylcellulose, glyceryl behenate, sodium alginate, calcium alginate, potassium alginate, hydroxypropyl methylcellulose (HPMC), carbomer, ammonium alginate, carnauba wax, tragacanth gum, arabic gum, and shellac;
the shaping material is selected from one or more of sodium carboxymethylcellulose, calcium carboxymethylcellulose, ethyl cellulose, carbomer, polylactic acid, a polylactic acid-glycolic acid copolymer, sodium alginate, hydrogenated vegetable oil, polymethacrylate, beeswax, glyceryl monostearate, polyethylene, and an ethylene-vinyl acetate copolymer;
the adhesive material is selected from one or more of hydroxyethyl cellulose, gelatin, polyoxyethylene, carbomer, pectin, chitosan, glucose, alginate, fucosamine, starch, and chitin; and
a blood drug concentration parameter achieved by the prolonged-release oral solid formulation when administered once a day to an individual at a daily dose is that: within 24 hours after an administration, a ratio of a maximum blood drug concentration (Cₘₐₓ) to a minimum blood drug concentration (Cₘᵢₙ) corresponding to a blood drug concentration curve is within 2-10, preferably within 2-5, and more preferably within 2-3.5.

2. The prolonged-release oral solid formulation of the pirfenidone according to claim 1, wherein a proportion of the sustained-release formulation to a total tablet weight is 50%-100%, or any value in the range; or the proportion of the sustained-release formulation to the total tablet weight is 50%, 60%, 65%, 70%, 73%, 78%, 80%, 85%, 90%, or a range formed by any two of these values.

3. The prolonged-release oral solid formulation of pirfenidone according to claim 2, wherein
an active pharmaceutical ingredient (API) in the sustained-release formulation accounts for 50%-100% of a total API, or any value in the range; or the proportion of the API in the sustained-release formulation in the total API is 50%, 60%, 65%, 70%, 73%, 78%, 85%, 73%, 80%, 85%, 90%, or a range formed by any two of these values.

4. The prolonged-release oral solid formulation of the pirfenidone according to claim 1, wherein a ratio of the swelling material to the adhesive material is 1-9:1 or any value in the range, or 8:1, 7:1, 6:1, 5:1, 4:1, 3:1, 2:1 or 1:1; and
a ratio of the adhesive material to the shaping material is (0.2-1) :1 or any value in the range, or 0.3:1, 0.4:1, 0.5:1, 0.6:1, 0.7:1, 0.8:1 or 0.9:1.

5. The prolonged-release oral solid formulation of the pirfenidone according to claim 2, wherein:
the prolonged-release oral solid formulation is a single-layer tablet, and the sustained-release formulation accounts for 100% of the weight of the single-layer tablet.

6. The prolonged-release oral solid formulation of the pirfenidone according to claim 2, wherein
the prolonged-release oral solid formulation further comprises an immediate-release formulation, and a ratio of the immediate-release formulation to the sustained-release formulation is 1-10, and preferably 2-8.

7. The prolonged-release oral solid formulation of the pirfenidone according to claim 6, wherein a ratio of an API in the immediate-release formulation to an API in the sustained-release formulation is 0.1-0.6.

8. The prolonged-release oral solid formulation of the pirfenidone according to claim 1, wherein
the viscosity of the swelling material is selected from any of the following ranges: 500 mpa.s - 5,000 mpa.s, 500 mpa.s-1,000 mpa.s, 2,000 mpa.s -5,000 mpa.s, 50,000 mpa.s -150,000 mpa.s or 150,000 mpa.s -300,000 mpa.s;
the viscosity of the shaping material is selected from any of the following ranges: 55 mpa.s - 60,000 mpa.s, 55 mpa.s -10,000 mpa.s, 55 mpa.s -1,000 mpa.s, or 60 mpa.s -200 mpa.s; and
the viscosity of the adhesive material is selected from any of the following ranges: 55 mpa.s - 100,000 mpa.s, 60 mpa.s -200 mpa.s, 65 mpa.s -10,000 mpa.s, 4,000 mpa.s -10,000 mpa.s, 5,000 mpa.s -10,000 mpa.s, 5,500 mpa.s -7,500 mpa.s or 7,000 mpa.s -10,000 mpa.s.

9. The prolonged-release oral solid formulation of pirfenidone according to claim 1, wherein
the swelling material employs the HPMC with a model selected from one or more of K100LV, K4M, K750, K100, K200M, and E5LV; and
a molecular weight of the polyoxyethylene is 100,000-7,000,000.

10. The prolonged-release oral solid formulation of the pirfenidone according to any one of claims 1-9, wherein the prolonged-release oral solid formulation is a tablet, and minimum sizes of the tablet in three dimensions of a length, a width and a height are not less than 8.0 mm;
the tablet with the minimum size in three dimension has an expansion growth rate satisfy the following conditions:
in 900 mL of a medium of pH 4.5 at 37°C, and at a rotation speed of 75 rpm;
the expansion growth rate within 1 h is ≥ 4.5%; the growth rate within 2 h is ≥ 12%; the expansion growth rate within 4 h is ≥ 25%; the growth rate within 6 h is ≥ 20%; and the expansion growth rate within 10 h is ≥ 40%.

11. The prolonged-release oral solid formulation of the pirfenidone according to claim 10, having any of the following specifications:
(1) a content of the pirfenidone is 500 mg-700 mg;
(2) the content of the pirfenidone is 600 mg;
(3) the content of the pirfenidone is 700 mg-1,000 mg; or
(4) the content of the pirfenidone is 801 mg.

12. The prolonged-release oral solid formulation of the pirfenidone according to any one of claims 1-9, wherein: according to a USP standard 2 method, in 900 mL of the medium of pH 4.5 at 37°C and at a rotation speed of 75 rpm, from the prolonged-release oral solid formulation more than 15% of an active substance is dissolved out within 0.5 h; more than 25% of the active substance is dissolved out within 2 h, 50-70% of the active substance is dissolved out within 10 h, and more than 80% of the active substance is dissolved out within 20 h.

13. The prolonged-release oral solid formulation of the pirfenidone according to claim 11, wherein when the prolonged-release oral solid formulation is used for the administration to an individual, the prolonged-release oral solid formulation has the following parameter characteristics: the prolonged-release oral solid formulation can stay in a stomach for at least 12 h; and within a single dosing interval of 24 h, a continuous time of a blood drug concentration being higher than 300 ng/mL is ≥ 15 h, and a Cₘₐₓ is not higher than 3,500 ng/mL, preferably the Cₘₐₓ is not higher than 2,500 ng/mL.

14. The prolonged-release oral solid formulation of pirfenidone according to claim 11, wherein when the prolonged-release oral solid formulation is used for administration to an individual, the blood drug concentration parameters comprise: the Cₘₐₓ of 800 ng/mL-3,500 ng/mL, and preferably 1,000ng/mL-2,500 ng/mL, and the Cₘᵢₙ ≥ 300 ng/mL and preferably the Cₘᵢₙ ≥ 400 ng/mL.

15. The prolonged-release oral solid formulation of pirfenidone according to claim 1, wherein the prolonged-release oral solid formulation is a tablet or a capsule, wherein the tablet is allowed to be a single-layer tablet, a double-layer tablet, a three-layer tablet, a core-coated tablet, or a ring-shaped tablet.

16. The prolonged-release oral solid formulation of the pirfenidone according to claim 1, wherein the prolonged-release oral solid formulation is a three-layer tablet, a middle layer is not less than 10% of a total weight of the tablet, and the third layer is not less than 15% of the total weight of the tablet.
